# EUROPEAN PATENT APPLICATION

(11) **EP 4 696 698 A1**
(43) Date of publication of application: **18.02.2026**
(21) Application number: 24788180.8
(22) Date of filing: 12.04.2024
(51) Int. Cl.: C07K 5/027, C07K 1/02, A61K 38/07, A61P 35/00

(54) **PREPARATION OF AND PURIFICATION METHOD FOR MONOMETHYL AURISTATIN E COMPOUND**

(30) Priority: 12.04.2023 CN 202310407223
(71) Applicant: RemeGen Co., Ltd., Shandong 264006 (CN)
(72) Inventor: ZHU, Marie M., Yantai, Shandong 264006 (CN); LI, Haitao, Yantai, Shandong 264006 (CN); LIANG, Xin, Yantai, Shandong 264006 (CN); LI, Xinli, Yantai, Shandong 264006 (CN); SUN, Xiaoqing, Yantai, Shandong 264006 (CN); SUN, Peng, Yantai, Shandong 264006 (CN); XIAO, Kai, Yantai, Shandong 264006 (CN); LIANG, Xiaowei, Yantai, Shandong 264006 (CN); GAO, Jinguo, Yantai, Shandong 264006 (CN)
(74) Representative: Maiwald GmbH
(86) International application number: PCT/CN2024/087383
(87) International publication number: WO 2024/213080

(57) **Abstract**

An MMAE preparation and purification method, that mainly replaces column chromatography purification in methods involved in the prior art with a purification method for toluene and n-heptane back-titration crystallization, so that the production efficiency is greatly improved, and the solvent consumption is effectively reduced. In addition, the MMAE prepared by the provided preparation and purification method is high in yield and purity, the production cost can be effectively reduced, the stability of the method is good, and the method is more suitable for large-scale production.

## Description

### FIELD

The present invention relates to the field of synthesis method of compounds, in particular to a method for producing and purifying a compound of monomethyl auristatin E (i.e., MMAE).

### BACKGROUND

Monomethyl auristantin E (MMAE, also known as methyl auristatin E), a fully synthetic derivative of auristatin, can effectively inhibit mitosis by inhibiting polymerization of tubulin. At present, it has been widely used as a cytotoxic small-molecule payload in the development of antibody-drug conjugates (abbreviated as ADCs) to treat cancer.

Chinese patent application No. CN105143199A discloses a method of preparing MMAE on page 29 of the specification:

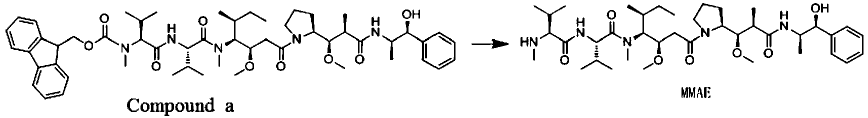

The method is as follows: dissolving the compound a in acetonitrile and piperidine under an inert atmosphere, stirring at ambient temperature overnight; concentrating under reduced pressure to dryness, and purifying the residue using a silica gel column with a mixture of dichloromethane and methanol to obtain MMAE as a white solid. The method gives a low yield of 68%. Besides, the purification step of the method involves silica gel column chromatography, which is costly and requires high-specification equipment and highly skilled operators. Moreover, the production efficiency is low due to the limited purification capacity of the silica gel column. Therefore, a synthesis and purification method with high yield, high purity, high efficiency and suitable for scale-up production is urgently needed.

### SUMMARY

To address the issues above, the present invention provides a method for producing and purifying MMAE which has a simple and efficient purification process and a high production efficiency, is environmentally friendly, and can provide a final product with a high quality (high purity and high yield).

Specifically, the present invention provides a method for producing and purifying a compound represented by formula (I) (i.e., MMAE) 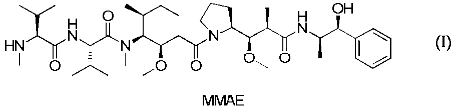 via a route of: 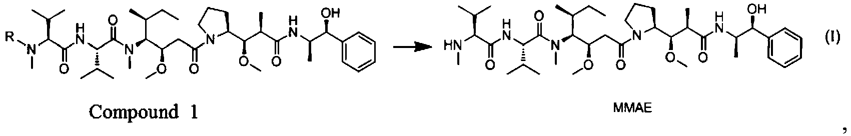 wherein R is an amino protecting group, and the method comprises steps of:
A. removing the amino protecting group R from compound 1 under alkaline condition,
B. completing a reaction in step A, then adding an appropriate amount of water to a reaction system of step A, stirring, filtering and collecting a filtrate,
C. adding an appropriate amount of a first organic solvent to the filtrate collected in step B, extracting and collecting an organic phase, concentrating the organic phase to obtain a concentrate a,
D. adding a second organic solvent to the concentrate a obtained in step C, dissolving to obtain a dissolved solution b, and
E. adding the dissolved solution b obtained in step D dropwise to a third organic solvent to allow precipitation of a large amount of solid, filtering by suction and collecting a filter cake to obtain MMAE.
   Wherein,
   the amino protecting group R is selected from the group consisting of Fmoc protecting group and trifluoroacetyl;
   the first organic solvent is selected from the group consisting of ethyl acetate, dichloromethane, isopropyl acetate, chloroform and methylbenzene;
   the second organic solvent is selected from the group consisting of methylbenzene, ethyl acetate and acetone; and
   the third organic solvent is selected from the group consisting of n-heptane, petroleum ether, n-hexane, cyclohexane, n-pentane, methylcyclohexane and methyl tert-butyl ether.

In some specific embodiments, the amino protecting group R has a structure selected from the group consisting of:

| | |
|---|---|
| Fmoc protecting group (9-fluorenylmethyloxycarbonyl) | 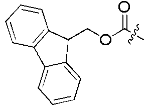 |
| Trifluoroacetyl | 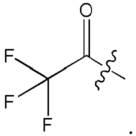 |

In some specific embodiments, the compound 1 has a structure selected from the group consisting of:

| | |
|---|---|
| 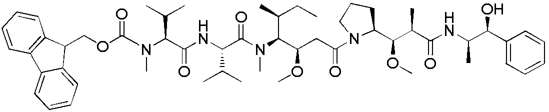 | Compound a |
| 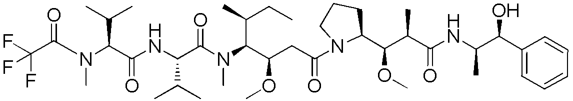 | Compound b. |

In some specific embodiments, the first organic solvent is ethyl acetate. Alternatively, in some other specific embodiments, the first organic solvent is dichloromethane. In some other specific embodiments, the first organic solvent is isopropyl acetate. In some other specific embodiments, the first organic solvent is chloroform. In some other specific embodiments, the first organic solvent is methylbenzene.

In some specific embodiments, the second organic solvent is methylbenzene. Alternatively, in some other specific embodiments, the second organic solvent is ethyl acetate. In some other specific embodiments, the second organic solvent is acetone.

In some specific embodiments, the third organic solvent is n-heptane. Alternatively, in some other specific embodiments, the third organic solvent is petroleum ether. In some other specific embodiments, the third organic solvent is n-hexane. In some other specific embodiments, the third organic solvent is cyclohexane. In some other specific embodiments, the third organic solvent is n-pentane. In some other specific embodiments, the third organic solvent is methylcyclohexane. In some other specific embodiments, the third organic solvent is methyl tert-butyl ether.

It can be understood that the first organic solvent, second organic solvent and third organic solvents are each selected independently, which means that the organic solvents involved in each step (the first organic solvent in step C, the second organic solvent in step D, the third organic solvent in step E) are each selected independently. In a relatively specific embodiment, the first organic solvent, second organic solvent and third organic solvent may be any one of the following combinations:

| Combination | First organic solvent | Second organic solvent | Third organic solvent |
|---|---|---|---|
| Combination 1 | Ethyl acetate | Methylbenzene | n-heptane |
| Combination 2 | Ethyl acetate | Methylbenzene | Petroleum ether |
| Combination 3 | Ethyl acetate | Methylbenzene | n-hexane |
| Combination 4 | Ethyl acetate | Methylbenzene | Cyclohexane |
| Combination 5 | Ethyl acetate | Methylbenzene | n-pentane |
| Combination 6 | Ethyl acetate | Methylbenzene | Methylcyclohexane, |
| Combination 7 | Ethyl acetate | Methylbenzene | Methyl tert-butyl ether |
| Combination 8 | Ethyl acetate | Ethyl acetate | n-heptane |
| Combination 9 | Ethyl acetate | Ethyl acetate | Petroleum ether |
| Combination 10 | Ethyl acetate | Ethyl acetate | n-hexane |
| Combination 11 | Ethyl acetate | Ethyl acetate | Cyclohexane |
| Combination 12 | Ethyl acetate | Ethyl acetate | n-pentane |
| Combination 13 | Ethyl acetate | Ethyl acetate | Methylcyclohexane, |
| Combination 14 | Ethyl acetate | Ethyl acetate | Methyl tert-butyl ether |
| Combination 15 | Ethyl acetate | Acetone | n-heptane |
| Combination 16 | Ethyl acetate | Acetone | Petroleum ether |
| Combination 17 | Ethyl acetate | Acetone | n-hexane |
| Combination 18 | Ethyl acetate | Acetone | Cyclohexane |
| Combination 19 | Ethyl acetate | Acetone | n-pentane |
| Combination 20 | Ethyl acetate | Acetone | Methylcyclohexane, |
| Combination 21 | Ethyl acetate | Acetone | Methyl tert-butyl ether |
| Combination 22 | Dichloromethane | Methylbenzene | n-heptane |
| Combination 23 | Dichloromethane | Methylbenzene | Petroleum ether |
| Combination 24 | Dichloromethane | Methylbenzene | n-hexane |
| Combination 25 | Dichloromethane | Methylbenzene | Cyclohexane |
| Combination 26 | Dichloromethane | Methylbenzene | n-pentane |
| Combination 27 | Dichloromethane | Methylbenzene | Methylcyclohexane, |
| Combination 28 | Dichloromethane | Methylbenzene | Methyl tert-butyl ether |
| Combination 29 | Dichloromethane | Ethyl acetate | n-heptane |
| Combination 30 | Dichloromethane | Ethyl acetate | Petroleum ether |
| Combination 31 | Dichloromethane | Ethyl acetate | n-hexane |
| Combination 32 | Dichloromethane | Ethyl acetate | Cyclohexane |
| Combination 33 | Dichloromethane | Ethyl acetate | n-pentane |
| Combination 34 | Dichloromethane | Ethyl acetate | Methylcyclohexane, |
| Combination 35 | Dichloromethane | Ethyl acetate | Methyl tert-butyl ether |
| Combination 36 | Dichloromethane | Acetone | n-heptane |
| Combination 37 | Dichloromethane | Acetone | Petroleum ether |
| Combination 38 | Dichloromethane | Acetone | n-hexane |
| Combination 39 | Dichloromethane | Acetone | Cyclohexane |
| Combination 40 | Dichloromethane | Acetone | n-pentane |
| Combination 41 | Dichloromethane | Acetone | Methylcyclohexane, |
| Combination 42 | Dichloromethane | Acetone | Methyl tert-butyl ether |
| Combination 43 | Isopropyl acetate | Methylbenzene | n-heptane |
| Combination 44 | Isopropyl acetate | Methylbenzene | Petroleum ether |
| Combination 45 | Isopropyl acetate | Methylbenzene | n-hexane |
| Combination 46 | Isopropyl acetate | Methylbenzene | Cyclohexane |
| Combination 47 | Isopropyl acetate | Methylbenzene | n-pentane |
| Combination 48 | Isopropyl acetate | Methylbenzene | Methylcyclohexane, |
| Combination 49 | Isopropyl acetate | Methylbenzene | Methyl tert-butyl ether |
| Combination 50 | Isopropyl acetate | Ethyl acetate | n-heptane |
| Combination 51 | Isopropyl acetate | Ethyl acetate | Petroleum ether |
| Combination 52 | Isopropyl acetate | Ethyl acetate | n-hexane |
| Combination 53 | Isopropyl acetate | Ethyl acetate | Cyclohexane |
| Combination 54 | Isopropyl acetate | Ethyl acetate | n-pentane |
| Combination 55 | Isopropyl acetate | Ethyl acetate | Methylcyclohexane, |
| Combination 56 | Isopropyl acetate | Ethyl acetate | Methyl tert-butyl ether |
| Combination 57 | Isopropyl acetate | Acetone | n-heptane |
| Combination 58 | Isopropyl acetate | Acetone | Petroleum ether |
| Combination 59 | Isopropyl acetate | Acetone | n-hexane |
| Combination 60 | Isopropyl acetate | Acetone | Cyclohexane |
| Combination 61 | Isopropyl acetate | Acetone | n-pentane |
| Combination 62 | Isopropyl acetate | Acetone | Methylcyclohexane, |
| Combination 63 | Isopropyl acetate | Acetone | Methyl tert-butyl ether |
| Combination 64 | Chloroform | Methylbenzene | n-heptane |
| Combination 65 | Chloroform | Methylbenzene | Petroleum ether |
| Combination 66 | Chloroform | Methylbenzene | n-hexane |
| Combination 67 | Chloroform | Methylbenzene | Cyclohexane |
| Combination 68 | Chloroform | Methylbenzene | n-pentane |
| Combination 69 | Chloroform | Methylbenzene | Methylcyclohexane |
| Combination 70 | Chloroform | Methylbenzene | Methyl tert-butyl ether |
| Combination 71 | Chloroform | Ethyl acetate | n-heptane |
| Combination 72 | Chloroform | Ethyl acetate | Petroleum ether |
| Combination 73 | Chloroform | Ethyl acetate | n-hexane |
| Combination 74 | Chloroform | Ethyl acetate | Cyclohexane |
| Combination 75 | Chloroform | Ethyl acetate | n-pentane |
| Combination 76 | Chloroform | Ethyl acetate | Methylcyclohexane, |
| Combination 77 | Chloroform | Ethyl acetate | Methyl tert-butyl ether |
| Combination 78 | Chloroform | Acetone | n-heptane |
| Combination 79 | Chloroform | Acetone | Petroleum ether |
| Combination 80 | Chloroform | Acetone | n-hexane |
| Combination 81 | Chloroform | Acetone | Cyclohexane |
| Combination 82 | Chloroform | Acetone | n-pentane |
| Combination 83 | Chloroform | Acetone | Methylcyclohexane |
| Combination 84 | Chloroform | Acetone | Methyl tert-butyl ether |
| Combination 85 | Methylbenzene | Methylbenzene | n-heptane |
| Combination 86 | Methylbenzene | Methylbenzene | Petroleum ether |
| Combination 87 | Methylbenzene | Methylbenzene | n-hexane |
| Combination 88 | Methylbenzene | Methylbenzene | Cyclohexane |
| Combination 89 | Methylbenzene | Methylbenzene | n-pentane |
| Combination 90 | Methylbenzene | Methylbenzene | Methylcyclohexane, |
| Combination 91 | Methylbenzene | Methylbenzene | Methyl tert-butyl ether |
| Combination 92 | Methylbenzene | Ethyl acetate | n-heptane |
| Combination 93 | Methylbenzene | Ethyl acetate | Petroleum ether |
| Combination 94 | Methylbenzene | Ethyl acetate | n-hexane |
| Combination 95 | Methylbenzene | Ethyl acetate | Cyclohexane |
| Combination 96 | Methylbenzene | Ethyl acetate | n-pentane |
| Combination 97 | Methylbenzene | Ethyl acetate | Methylcyclohexane, |
| Combination 98 | Methylbenzene | Ethyl acetate | Methyl tert-butyl ether |
| Combination 99 | Methylbenzene | Acetone | n-heptane |
| Combination 100 | Methylbenzene | Acetone | Petroleum ether |
| Combination 101 | Methylbenzene | Acetone | n-hexane |
| Combination 102 | Methylbenzene | Acetone | Cyclohexane |
| Combination 103 | Methylbenzene | Acetone | n-pentane |
| Combination 104 | Methylbenzene | Acetone | Methylcyclohexane |
| Combination 105 | Methylbenzene | Acetone | Methyl tert-butyl ether |

In some specific embodiments, the water in step B is pure water.

In some specific embodiments, the dissolving in step D is carried out under stirring.

In some preferred embodiments, a weight-to-volume ratio (g/ml) of the compound 1 in step A to pure water in step B is 1:4 to 1:20. In some specific embodiments, a weight-to-volume ratio (g/ml) of the compound 1 in step A to pure water in step B is 1:4, 1:5, 1:6, 1:7, 1:8, 1:9, 1:10, 1:11, 1:12, 1:13, 1:14, 1:15, 1:16, 1:17, 1:18, 1:19 or 1:20. In some other specific embodiments, a weight-to-volume ratio of the compound 1 in step A to pure water in step B may also be non-limiting other ratios within the range, such as 1:14.1, 1:14.2, 1:14.3, 1:14.4 and 1:14.5.

In some preferred embodiments, the stirring in step B is carried out at a low temperature. In some more preferred embodiments, the stirring in step B is carried out at a low temperature of 0°C to 10°C. In some specific embodiments, the stirring in step B is carried out at a low temperature of 0°C, 1°C, 2°C, 3°C, 4°C, 5°C, 6°C, 7°C, 8°C, 9°C or 10°C. Alternatively, in some specific embodiments, the stirring in step B can be carried out at any other low temperature within the range. Alternatively, in some specific embodiments, the stirring in step B can be carried out at a low temperature of 0°C, 1°C, 2°C, 3°C, 4°C, 5°C, 6°C, 7°C, 8°C, 9°C or 10°C, with fluctuation of 1°C to 5°C up or down, such as -5°C, -4°C, -3°C, -2°C, -1°C, 11°C, 12°C, 13°C, 14°C and 15°C.

In some preferred embodiments, a weight-to-volume ratio (g/ml) of the compound 1 in step A to the first organic solvent in step C is 1:5 to 1:25. In some specific embodiments, a weight-to-volume ratio (g/ml) of the compound 1 in step A to the first organic solvent in step C is 1:5, 1:6, 1:7, 1:8, 1:9, 1:10, 1:11, 1:12, 1:13, 1:14, 1:15, 1:16, 1:17, 1:18, 1:19, 1:20, 1:21, 1:22, 1:23, 1:24 or 1:25. In some other specific embodiments, a weight-to-volume ratio of the compound 1 in step A to the first organic solvent in step C may be any other ratio within the range.

In some more preferred embodiments, a weight-to-volume ratio (g/ml) of the compound 1 in step A to the first organic solvent in step C is 1:5 to 1:15. In some specific embodiments, a weight-to-volume ratio (g/ml) of the compound 1 in step A to the first organic solvent in step C is 1:5, 1:6, 1:7, 1:8, 1:9, 1:10, 1:11, 1:12, 1:13, 1:14 or 1:15. Alternatively, in some other specific embodiments, a weight-to-volume ratio of the compound 1 in step A to the first organic solvent in step C may be any other ratio within the range.

In some preferred embodiments, a weight-to-volume ratio (g/ml) of the concentrate a in step C to the second organic solvent in step D is 1:3 to 1:15. In some specific embodiments, a weight-to-volume ratio (g/ml) of the concentrate a in step C to the second organic solvent in step D is 1:3, 1:4, 1:5, 1:6, 1:7, 1:8, 1:9, 1:10, 1:11, 1:12, 1:13, 1:14 or 1:15. In some other specific embodiments, a weight-to-volume ratio of the concentrate a in step C to the second organic solvent in step D may also be any other ratio within the above-mentioned range.

In some more preferred embodiments, a weight-to-volume ratio (g/ml) of the concentrate a in step C to the second organic solvent in step D is 1:5 to 1:10. In some specific embodiments, a weight-to-volume ratio (g/ml) of the concentrate a in step C to the second organic solvent in step D is 1:5, 1:6, 1:7, 1:8, 1:9 or 1:10. In some other specific embodiments, a weight-to-volume ratio of the concentrate a in step C to the second organic solvent in step D may also be any other ratio within the above-mentioned range.

In some preferred embodiments, a weight-to-volume ratio (g/ml) of the concentrate a in step C to the third organic solvent in step E is 1:15 to 1:45. In some specific embodiments, a weight-to-volume ratio (g/ml) of the concentrate a in step C to the third organic solvent in step E is 1:15, 1:16, 1:17, 1:18, 1:19, 1:20, 1:21, 1:22, 1:23, 1:24, 1:25, 1:26, 1:27, 1:28, 1:29, 1:30, 1:31, 1:32, 1:33, 1:34, 1:35, 1:36, 1:37, 1:38, 1:39, 1:40, 1:41, 1:42, 1:43, 1:44 or 1:45. Alternatively, in some other specific embodiments, a weight-to-volume ratio of the concentrate a in step C to the third organic solvent in step E may be any other ratio within the above-mentioned range.

In some more preferred embodiments, a weight-to-volume ratio (g/ml) of the concentrate a in step C to the third organic solvent in step E is 1:20 to 1:40. In some specific embodiments, a weight-to-volume ratio (g/ml) of the concentrate a in step C to the third organic solvent in step E is 1:20, 1:21, 1:22, 1:23, 1:24, 1:25, 1:26, 1:27, 1:28, 1:29, 1:30, 1:31, 1:32, 1:33, 1:34, 1:35, 1:36, 1:37, 1:38, 1:39 or 1:40. Alternatively, in some other specific embodiments, a weight-to-volume ratio of the concentrate a in step C to the third organic solvent in step E may be any other ratio within the above-mentioned range.

Alternatively, in some more preferred embodiments, a weight-to-volume ratio (g/ml) of the concentrate a in step C to the third organic solvent in step E is 1:25 to 1:35. In some other specific embodiments, a weight-to-volume ratio (g/ml) of the concentrate a in step C to the third organic solvent in step E is 1:25, 1:26, 1:27, 1:28, 1:29, 1:30, 1:31, 1:32, 1:33, 1:34 or 1:35. Alternatively, in some other specific embodiments, a weight-to-volume ratio of the concentrate a in step C to the third organic solvent in step E may be any other ratio within the above-mentioned range.

It can be understood that the concentrate a in step C is actually unpurified MMAE, and the concentrating in step C is concentration under reduced pressure, i.e., concentrating the collected organic phase to a certain degree under reduced pressure. In some preferred embodiments, step C is carried out by concentrating the collected organic phase to dryness under reduced pressure to obtain the concentrate a as a solid.

In some preferred embodiments, an alkali used in the step of removing the amino protecting group R under alkaline condition includes, but is not limited to piperidine, diethylamine and DBU. In some specific embodiments, the amino protecting group is removed from the compound 1 using piperidine. In some other specific embodiments, the amino protecting group is removed from the compound 1 using diethylamine. Alternatively, in some other specific embodiments, the amino protecting group is removed from the compound 1 using DBU.

In some specific embodiments, the amino protecting group is removed from the compound 1 using piperidine. Specifically, the process is, without limitation, carried out by dissolving the compound 1 in an appropriate amount of a fourth organic solvent (the fourth organic solvent can be, without limitation, selected from the group consisting of acetonitrile, ethanol and methanol) under stirring, and adding piperidine to initiate a reaction. In some preferred embodiments, a weight-to-volume ratio (g/ml) of the compound 1 to the fourth organic solvent is 1:2 to 1:12 (for example 1:2, 1:3, 1:4, 1:5, 1:6, 1:7, 1:8, 1:9, 1:10, 1:11 or 1:12, or other non-integer ratios such as 1:2.4, 1:2.6, 1:2.8,..., 1:3.7,..., 1:5.5,..., 1:10.4,...etc.). An equivalence ratio of the compound 1 to the piperidine is 1:2 to 1:8 (for example 1:2, 1:3, 1:4, 1:5, 1:6, 1:7 or 1:8, or other non-integer ratios such as 1:2.4, 1:2.6, 1:2.8,..., 1:3.7,..., 1:5.5,...etc.). In some more specific embodiments, the specific process of removing the amino protecting group from the compound 1 using piperidine is, without limitation, carried out by dissolving the compound 1 in an appropriate amount of acetonitrile under stirring, and adding piperidine to initiate a reaction, with an equivalence ratio of the compound 1 to the piperidine of 1:3. After the reaction is completed, the next reaction is carried out. In some other more specific embodiments, the specific process of removing the amino protecting group from the compound 1 using piperidine is, without limitation, carried out by dissolving the compound 1 in an appropriate amount of methanol under stirring, and adding piperidine to initiate a reaction, with an equivalence ratio of the compound 1 to the piperidine of 1:4. After the reaction is completed, the next reaction is carried out. Alternatively, in yet some other more specific embodiments, the specific process of removing the amino protecting group from the compound 1 using piperidine is, without limitation, carried out by dissolving the compound 1 in an appropriate amount of ethanol under stirring, and adding piperidine to initiate a reaction, with an equivalence ratio of the compound 1 to the piperidine of 1:5. After the reaction is completed, the next reaction is carried out.

In some other specific embodiments, the amino protecting group is removed from the compound 1 using diethylamine. Specifically, this process is, without limitation, carried out by dissolving the compound 1 in an appropriate amount of a fourth organic solvent (the fourth organic solvent can be, without limitation, selected from the group consisting of acetonitrile, ethanol and methanol) under stirring, and adding piperidine to initiate a reaction. In some preferred embodiments, a weight-to-volume ratio (g/ml) of the compound 1 to the fourth organic solvent is 1:2 to 1:12 (for example 1:2, 1:3, 1:4, 1:5, 1:6, 1:7, 1:8, 1:9, 1:10, 1:11 or 1:12, or other non-integer ratios such as 1:2.4, 1:2.6, 1:2.8,..., 1:3.7,..., 1:5.5,..., 1:10.4,...etc.). An equivalence ratio of the compound 1 to the diethylamine is 1:2 to 1:8 (for example 1:2, 1:3, 1:4, 1:5, 1:6, 1:7 or 1:8, or other non-integer ratios such as 1:2.4, 1:2.6, 1:2.8,..., 1:3.7,..., 1:5.5,...etc.). In some more specific embodiments, the specific process of removing the amino protecting group from the compound 1 using diethylamine is, without limitation, carried out by dissolving the compound 1 in an appropriate amount of methanol under stirring, and adding diethylamine to initiate a reaction, with an equivalence ratio of the compound 1 to the diethylamine of 1:3. After the reaction is completed, the next reaction is carried out. In some more specific embodiments, the specific process of removing the amino protecting group from the compound 1 using diethylamine is , without limitation, carried out by dissolving the compound 1 in an appropriate amount of ethanol under stirring, and adding diethylamine to initiate a reaction, with an equivalence ratio of the compound 1 to the diethylamine of 1:3. After the reaction is completed, the next reaction is carried out. In some other more specific embodiments, the specific process of removing the amino protecting group from the compound 1 using diethylamine is, without limitation, carried out by dissolving the compound 1 in an appropriate amount of acetonitrile under stirring, and adding diethylamine to initiate a reaction, with an equivalence ratio of the compound 1 to the diethylamine of 1:4. After the reaction is completed, the next reaction is carried out.

In some other specific embodiments, the amino protecting group is removed from the compound 1 using DBU. Specifically, this process is, without limitation, carried out by dissolving the compound 1 in an appropriate amount of a fourth organic solvent (the fourth organic solvent can be, without limitation, selected from the group consisting of acetonitrile, ethanol and methanol) under stirring, and adding piperidine to initiate a reaction. In some preferred embodiments, a weight-to-volume ratio (g/ml) of the compound 1 to the fourth organic solvent is 1:2 to 1:12 (for example 1:2, 1:3, 1:4, 1:5, 1:6, 1:7, 1:8, 1:9, 1:10, 1:11 or 1:12, or other non-integer ratios such as 1:2.4, 1:2.6, 1:2.8,..., 1:3.7,..., 1:5.5,..., 1:10.4,...etc.). An equivalence ratio of the compound 1 to the DBU is 1:2 to 1:8 (for example 1:2, 1:3, 1:4, 1:5, 1:6, 1:7 or 1:8, or other non-integer ratios such as 1:2.4, 1:2.6, 1:2.8,..., 1:3.7,..., 1:5.5,...etc.). In some more specific embodiments, the specific process of removing the amino protecting group from the compound 1 using DBU is, without limitation, carried out by dissolving the compound 1 in an appropriate amount of methanol under stirring, and adding DBU to initiate a reaction, with an equivalence ratio of the compound 1 to the DBU of 1:3. After the reaction is completed, the next reaction is carried out. In some more specific embodiments, the specific process of removing the amino protecting group from the compound 1 using DBU is, without limitation, carried out by dissolving the compound 1 in an appropriate amount of ethanol under stirring, and adding DBU to initiate a reaction, with an equivalence ratio of the compound 1 to the DBU of 1:3. After the reaction is completed, the next reaction is carried out. In some other more specific embodiments, the specific process of removing the amino protecting group from the compound 1 using DBU is, without limitation, carried out by dissolving the compound 1 in an appropriate amount of acetonitrile under stirring, and adding DBU to initiate a reaction, with an equivalence ratio of the compound 1 to the DBU of 1:4. After the reaction is completed, the next reaction is carried out.

The present invention further provides use of a reverse dropwise addition method in the production and purification of MMAE via a route of 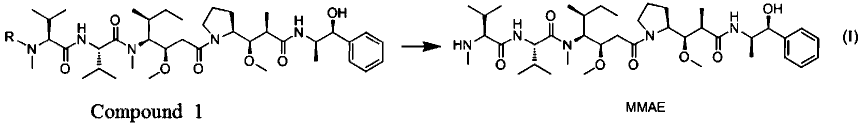 wherein R is an amino protecting group,
the reverse dropwise addition method comprises dissolving a reaction product produced by the route, which is to be purified and comprises MMAE, in a second organic solvent to obtain a solution, reversely adding the solution dropwise to a third organic solvent, filtering and drying to obtain purified MMAE;
wherein, the second organic solvent is selected from the group consisting of methylbenzene, ethyl acetate and acetone; and
the third organic solvent is selected from the group consisting of n-heptane, petroleum ether, n-hexane, cyclohexane, n-pentane, methylcyclohexane and methyl tert-butyl ether.

The reverse dropwise addition method comprises steps of:
(1) dissolving the reaction product comprising MMAE to be purified in a second organic solvent to obtain a solution 1,
(2) adding the solution 1 dropwise to a third organic solvent to allow precipitation of a large amount of solid, filtering by suction and collecting a filter cake to obtain purified MMAE.

The above-mentioned term "reaction product produced by the route comprising MMAE to be purified" or "reaction product comprising MMAE to be purified" means, as the name suggests, unpurified crude MMAE produced by the above-mentioned route. For example, in some specific embodiments of the present invention, the term "reaction product produced by the route comprising MMAE to be purified" or "reaction product comprising MMAE to be purified" refers to a crude product obtained by removing the amino protecting group from compound 1, filtering, extracting and concentrating under reduced pressure, i.e., concentrate a.

The above-mentioned "reverse dropwise addition method" involves a procedure in contrast to the conventional organic experimental procedure of adding a solvent into a solution containing the product. Specifically, in the present invention, it means dissolving the reaction product produced by the route comprising MMAE to be purified in a second organic solvent to obtain a solution, and then reversely adding the solution dropwise to a third organic solvent.

In some preferred embodiments, a weight-to-volume ratio (g/ml) of the reaction product comprising MMAE to be purified in step (1) to the second organic solvent is 1:3 to 1:15. Preferably, the weight-to-volume ratio (g/ml) of the reaction product comprising MMAE to be purified in step (1) to the second organic solvent is 1:5 to 1:10.

In some preferred embodiments, a weight-to-volume ratio (g/ml) of the reaction product comprising MMAE to be purified in step (1) to the third organic solvent in step (2) is 1:15 to 1:45. Preferably, the weight-to-volume ratio (g/ml) of the reaction product comprising MMAE to be purified in step (1) to the third organic solvent in step (2) is 1:20 to 1:40. Preferably, a weight-to-volume ratio (g/ml) of the reaction product comprising MMAE to be purified in step (1) to the third organic solvent in step (2) is 1:25 to 1:35.

In some preferred embodiments, the weight-to-volume ratio (g/ml) of the reaction product comprising MMAE to be purified in step (1) to the second organic solvent is 1:3 to 1:15. In some specific embodiments, the weight-to-volume ratio (g/ml) of the reaction product comprising MMAE to be purified in step (1) to the second organic solvent is 1:3, 1:4, 1:5, 1:6, 1:7, 1:8, 1:9, 1:10, 1:11, 1:12, 1:13, 1:14 or 1:15. In some other specific embodiments, the weight-to-volume ratio of the reaction product comprising MMAE to be purified in step (1) to the second organic solvent may be any other ratio within the above-mentioned range.

In some more preferred embodiments, the weight-to-volume ratio (g/ml) of the reaction product comprising MMAE to be purified in step (1) to the second organic solvent is 1:5 to 1:10. In some specific embodiments, the weight-to-volume ratio (g/ml) of the reaction product comprising MMAE to be purified in step (1) to the second organic solvent is 1:5, 1:6, 1:7, 1:8, 1:9 or 1:10. In some other specific embodiments, the weight-to-volume ratio of the reaction product comprising MMAE to be purified in step (1) to the second organic solvent may be any other ratio within the above-mentioned range.

In some preferred embodiments, the weight-to-volume ratio (g/ml) of the reaction product comprising MMAE to be purified in step (1) to the third organic solvent in step (2) is 1:15 to 1:45. In some specific embodiments, the weight-to-volume ratio (g/ml) of the reaction product comprising MMAE to be purified in step (1) to the third organic solvent in step (2) is 1:15, 1:16, 1:17, 1:18, 1:19, 1:20, 1:21, 1:22, 1:23, 1:24, 1:25, 1:26, 1:27, 1:28, 1:29, 1:30, 1:31, 1:32, 1:33, 1:34, 1:35, 1:36, 1:37, 1:38, 1:39, 1:40, 1:41, 1:42, 1:43, 1:44 or 1:45. Alternatively, in some other specific embodiments, the weight-to-volume ratio of the reaction product comprising MMAE to be purified in step (1) to the third organic solvent in step (2) may be any other ratio within the above-mentioned range.

In some more preferred embodiments, the weight-to-volume ratio (g/ml) of the reaction product comprising MMAE to be purified in step (1) to the third organic solvent in step (2) is 1:20 to 1:40. In some specific embodiments, the weight-to-volume ratio (g/ml) of the reaction product comprising MMAE to be purified in step (1) to the third organic solvent in step (2) is 1:20, 1:21, 1:22, 1:23, 1:24, 1:25, 1:26, 1:27, 1:28, 1:29, 1:30, 1:31, 1:32, 1:33, 1:34, 1:35, 1:36, 1:37, 1:38, 1:39 or 1:40. Alternatively, in some other specific embodiments, the weight-to-volume ratio of the reaction product comprising MMAE to be purified in step (1) to the third organic solvent in step (2) may be any other ratio within the above-mentioned range.

Alternatively, in some more preferred embodiments, the weight-to-volume ratio (g/ml) of the reaction product comprising MMAE to be purified in step (1) to the third organic solvent in step (2) is 1:25 to 1:35. In some other specific embodiments, the weight-to-volume ratio (g/ml) of the reaction product comprising MMAE to be purified in step (1) to the third organic solvent in step (2) is 1:25, 1:26, 1:27, 1:28, 1:29, 1:30, 1:31, 1:32, 1:33, 1:34 or 1:35. Alternatively, in some other specific embodiments, the weight-to-volume ratio of the reaction product comprising MMAE to be purified in step (1) to the third organic solvent in step (2) may be any other ratio within the above-mentioned range.

The present invention further provides use of a reverse dropwise addition method in the purification of MMAE. The reverse dropwise addition method involves dissolving a reaction product comprising MMAE to be purified in a second organic solvent to obtain a solution, reversely adding the solution dropwise to a third organic solvent, filtering and drying to obtain purified MMAE.

Wherein, the second organic solvent is selected from the group consisting of methylbenzene, ethyl acetate and acetone; and
the third organic solvent is selected from the group consisting of n-heptane, petroleum ether, n-hexane, cyclohexane, n-pentane, methylcyclohexane and methyl tert-butyl ether.

In some preferred embodiments, the reverse dropwise addition method comprises steps of:
(a) dissolving the reaction product comprising MMAE to be purified in a second organic solvent to obtain a solution 2,
(b) adding the solution 2 dropwise to a third organic solvent to allow precipitation of a large amount of solid, filtering by suction and collecting a filter cake to obtain purified MMAE.

In some preferred embodiments, a weight-to-volume ratio (g/ml) of the reaction product comprising MMAE to be purified in step (a) to the second organic solvent is 1:3 to 1:15. Preferably, the weight-to-volume ratio (g/ml) of the reaction product comprising MMAE to be purified in step (a) to the second organic solvent is 1:5 to 1:10.

In some preferred embodiments, a weight-to-volume ratio (g/ml) of the reaction product comprising MMAE to be purified in step (a) to the third organic solvent in step (b) is 1:15 to 1:45. Preferably, the weight-to-volume ratio (g/ml) of the reaction product comprising MMAE to be purified in step (a) to the third organic solvent in step (b) is 1:20 to 1:40. Preferably, the weight-to-volume ratio (g/ml) of the reaction product comprising MMAE to be purified in step (a) to the third organic solvent in step (b) is 1:25 to 1:35.

In some preferred embodiments, the weight-to-volume ratio (g/ml) of the reaction product comprising MMAE to be purified in step (a) to the second organic solvent is 1:3 to 1:15. In some specific embodiments, the weight-to-volume ratio (g/ml) of the reaction product comprising MMAE to be purified in step (a) to the second organic solvent is 1:3, 1:4, 1:5, 1:6, 1:7, 1:8, 1:9, 1:10, 1:11, 1:12, 1:13, 1:14 or 1:15. In some other specific embodiments, the weight-to-volume ratio of the reaction product comprising MMAE to be purified in step (a) to the second organic solvent may be any other ratio within the above-mentioned range.

In some more preferred embodiments, the weight-to-volume ratio (g/ml) of the reaction product comprising MMAE to be purified in step (a) to the second organic solvent is 1:5 to 1:10. In some specific embodiments, the weight-to-volume ratio (g/ml) of the reaction product comprising MMAE to be purified in step (a) to the second organic solvent is 1:5, 1:6, 1:7, 1:8, 1:9 or 1:10. In some other specific embodiments, the weight-to-volume ratio of the reaction product comprising MMAE to be purified in step (a) to the second organic solvent may be any other ratio within the above-mentioned range.

In some more preferred embodiments, the weight-to-volume ratio (g/ml) of the reaction product comprising MMAE to be purified in step (a) to the third organic solvent in step (b) is 1:15 to 1:45. In some specific embodiments, the weight-to-volume ratio (g/ml) of the reaction product comprising MMAE to be purified in step (a) to the third organic solvent in step (b) is 1:15, 1:16, 1:17, 1:18, 1:19, 1:20, 1:21, 1:22, 1:23, 1:24, 1:25, 1:26, 1:27, 1:28, 1:29, 1:30, 1:31, 1:32, 1:33, 1:34, 1:35, 1:36, 1:37, 1:38, 1:39, 1:40, 1:41, 1:42, 1:43, 1:44 or 1:45. Alternatively, in some other specific embodiments, the weight-to-volume ratio of the reaction product comprising MMAE to be purified in step (a) to the third organic solvent in step (b) may be any other ratio within the above-mentioned range.

In some more preferred embodiments, the weight-to-volume ratio (g/ml) of the reaction product comprising MMAE to be purified in step (a) to the third organic solvent in step (b) is 1:20 to 1:40. In some specific embodiments, the weight-to-volume ratio (g/ml) of the reaction product comprising MMAE to be purified in step (a) to the third organic solvent in step (b) is 1:20, 1:21, 1:22, 1:23, 1:24, 1:25, 1:26, 1:27, 1:28, 1:29, 1:30, 1:31, 1:32, 1:33, 1:34, 1:35, 1:36, 1:37, 1:38, 1:39 or 1:40. Alternatively, in some other specific embodiments, the weight-to-volume ratio of the reaction product comprising MMAE to be purified in step (a) to the third organic solvent in step (b) may be any other ratio with the range above-mentioned.

Alternatively, in some more preferred embodiments, the weight-to-volume ratio (g/ml) of the reaction product comprising MMAE to be purified in step (a) to the third organic solvent in step (b) is 1:25 to 1:35. In some other specific embodiments, the weight-to-volume ratio (g/ml) of the reaction product comprising MMAE to be purified in step (a) to the third organic solvent in step (b) is 1:25, 1:26, 1:27, 1:28, 1:29, 1:30, 1:31, 1:32, 1:33, 1:34 or 1:35. Alternatively, in some other specific embodiments, the weight-to-volume ratio of the reaction product comprising MMAE to be purified in step (a) to the third organic solvent in step (b) may be any other ratio within the above-mentioned range.

Provided is use of the above method of producing and/or purifying MMAE in the manufacture of an antibody drug conjugate containing MMAE as a toxin.

Provided is use of the above method of producing and/or purifying MMAE in the manufacture of an intermediate of an antibody drug conjugate containing MMAE as a toxin.

The method of producing and/or purifying MMAE provided by the present invention replaces the column chromatography adopted by methods in the prior art with the purification method of reverse dropwise addition for precipitation using methylbenzene and n-heptane, greatly improving the production efficiency, effectively reducing the amount of solvent used, and reducing the production cost. In addition, the production and purification method provided by the present invention can produce MMAE with a significantly improved yield and purity, has good stability, and is suitable for scaled-up production.

### BRIEF DESCRIPTION OF DRAWINGS

FIG. 1 is a chromatogram of MMAE produced and purified by the method provided in Example 1.
FIG. 2 is a chromatogram of MMAE produced and purified by the method provided in Example 2.
FIG. 3 is a chromatogram of MMAE produced and purified by the method provided in Example 3.
FIG. 4 is a chromatogram of MMAE produced and purified by the method provided in Example 4.
FIG. 5 is a chromatogram of MMAE produced and purified by the method provided in Example 5.
FIG. 6 is a chromatogram of MMAE produced and purified by the method provided in Example 6.
FIG. 7 is a chromatogram of MMAE produced and purified by the method provided in Example 7.
FIG. 8 is a chromatogram of MMAE produced and purified by the method provided in Example 8.
FIG. 9 is a chromatogram of MMAE produced and purified by the method provided in Example 9.
FIG. 10 is a chromatogram of MMAE produced and purified by the method provided in Example 10.
FIG. 11 is a chromatogram of MMAE produced and purified by the method provided in Example 11.
FIG. 12 is a chromatogram of MMAE produced and purified by the method provided in Example 12.
FIG. 13 is a chromatogram of MMAE produced and purified by the method provided in Example 13.
FIG. 14 is a chromatogram of MMAE produced and purified by the method provided in Example 14.
FIG. 15 is a chromatogram of MMAE produced and purified by the method provided in Example 15.
FIG. 16 is a chromatogram of MMAE produced and purified by the method provided in Example 16.
FIG. 17 is a chromatogram of MMAE produced and purified by the method provided in Comparative example 1.
FIG. 18 is a chromatogram of MMAE produced and purified by the method provided in Comparative example 2.

### DETAILED DESCRIPTION

The technical solutions of the present invention will be further described in detail, without limitation, in conjunction with specific embodiments below. It should be noted that the following examples are only to illustrate the technical concept and features of the present invention, and the purpose thereof is to enable those skilled in the art to understand the content of the present invention and implement it accordingly, but not to limit the protection scope of the present invention. All equivalent changes or modifications made according to the spirit of the present invention should be encompassed within the protection scope of the present disclosure

### Example 1

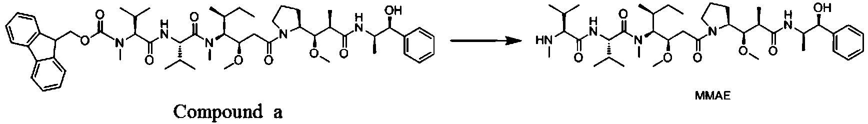

The Fmoc protecting group was removed from compound a by alkali method. Specifically, 100.0 g of compound a (i.e., Fmoc-MMAE, the same below) and 500 ml of acetonitrile were added to a three-necked flask, stirred and dissolved. 36.25 g of piperidine was then added for reaction.

After the reaction was completed, 500 ml of pure water was added to the reaction solution, and the reaction flask was placed in a low-temperature bath. The reaction solution was stirred at 0-5°C for 0.5 h and then filtered.

1000 ml of dichloromethane was added for extraction. The organic phase was separated and concentrated to dryness under reduced pressure at 20-25°C to obtain 71.03 g of concentrate (i.e., crude MMAE).

The concentrate (i.e., crude MMAE) was dissolved in 710 ml of methylbenzene and transferred to a constant pressure dropping funnel.

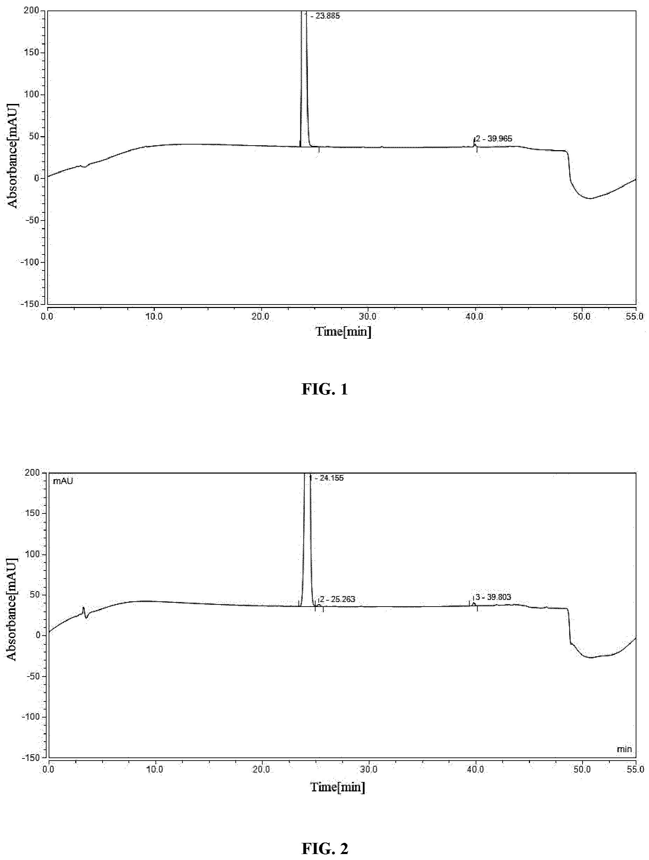

The solution of crude MMAE in methylbenzene was added dropwise to 2131 ml of n-heptane under stirring, a large amount of solids precipitated, and filtration by suction was carried out to obtain 67.6 g of purified MMAE, with a yield of 88.5%, a purity of 99.97%, and a single impurity of 0.03%. The chromatogram is shown in FIG. 1.

### Example 2

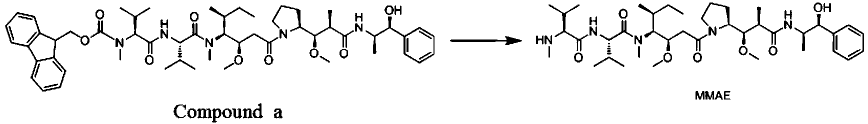

The Fmoc protecting group was removed from compound a by alkali method. Specifically, 2.0 g of compound a and 4 ml of acetonitrile were added to a three-necked flask, stirred and dissolved. 362 mg of piperidine was then added for reaction.

After the reaction was completed, 8 ml of pure water was added to the reaction solution, and the reaction flask was placed in a low-temperature bath. The reaction solution was stirred at 0-5°C for 0.5 h and then filtered.

10 ml of dichloromethane was added for extraction. The organic phase was separated and concentrated to dryness under reduced pressure at 20-25°C to obtain 1.40 g of concentrate (i.e., crude MMAE).

The concentrate (i.e., crude MMAE) was dissolved in 4.2 ml of methylbenzene and transferred to a constant pressure dropping funnel.

The solution of crude MMAE in methylbenzene was added dropwise to 21 ml of n-heptane under stirring, a large amount of solids precipitated, and filtration by suction was carried out to obtain 1.30 g of purified MMAE, with a yield of 83.7%, a purity of 99.60%, and a single impurity of 0.21%. The chromatogram is shown in FIG. 2.

### Example 3

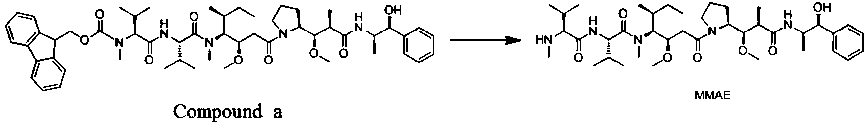

The Fmoc protecting group was removed from compound a by alkali method. Specifically, 2.0 g of compound a and 4 ml of acetonitrile were added to a three-necked flask, stirred and dissolved. 362 mg of piperidine was then added for reaction.

After the reaction was completed, 8 ml of pure water was added to the reaction solution, and the reaction flask was placed in a low-temperature bath. The reaction solution was stirred at 0-5°C for 0.5 h and then filtered.

10 ml of dichloromethane was added for extraction. The organic phase was separated and concentrated to dryness under reduced pressure at 20-25°C to obtain 1.42 g of concentrate (i.e., crude MMAE).

The concentrate (i.e., crude MMAE) was dissolved in 4.3 ml of methylbenzene and transferred to a constant pressure dropping funnel.

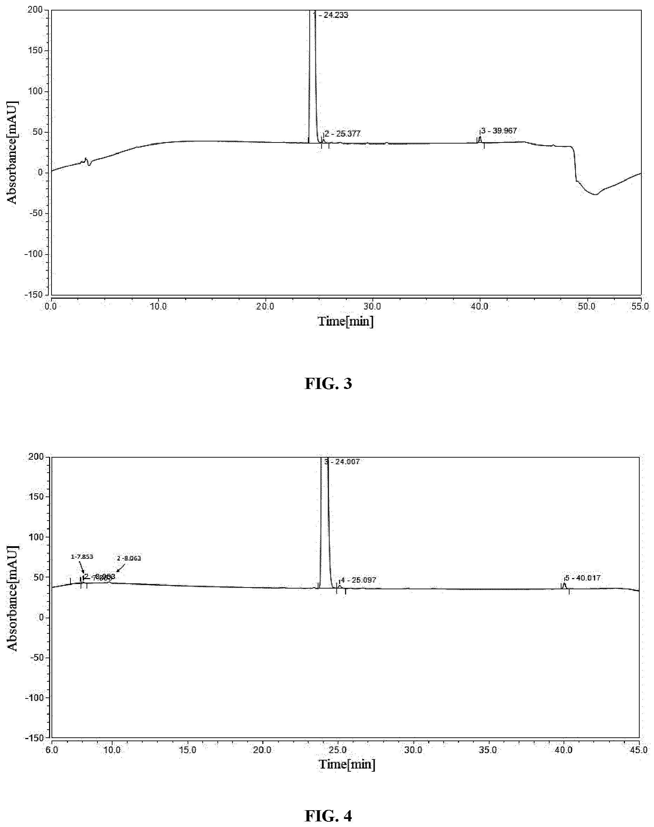

The solution of crude MMAE in methylbenzene was added dropwise to 28.4 ml of n-heptane under stirring, a large amount of solids precipitated, and filtration by suction was carried out to obtain 1.30 g of purified MMAE, with a yield of 85%, a purity of 99.58%, and a single impurity of 0.24%. The chromatogram is shown in FIG. 3.

### Example 4

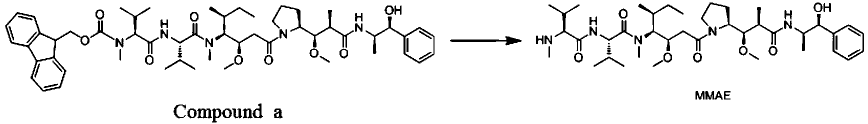

The Fmoc protecting group was removed from compound a by alkali method. Specifically, 2.0 g of compound a and 4 ml of acetonitrile were added to a three-necked flask, stirred and dissolved. 362 mg of piperidine was then added for reaction.

After the reaction was completed, 8 ml of pure water was added to the reaction solution, and the reaction flask was placed in a low-temperature bath. The reaction solution was stirred at 0-5°C for 0.5 h and then filtered.

10 ml of dichloromethane was added for extraction. The organic phase was separated and concentrated to dryness under reduced pressure at 20-25°C to obtain 1.44 g of concentrate (i.e., crude MMAE).

The concentrate (i.e., crude MMAE) was dissolved in 4.3 ml of methylbenzene and transferred to a constant pressure dropping funnel.

The solution of crude MMAE in methylbenzene was added dropwise to 36 ml of n-heptane under stirring, a large amount of solids precipitated, and filtration by suction was carried out to obtain 1.33 g of purified MMAE, with a yield of 86.9%, a purity of 99.21%, and a single impurity of 0.35%. The chromatogram is shown in FIG. 4.

### Example 5

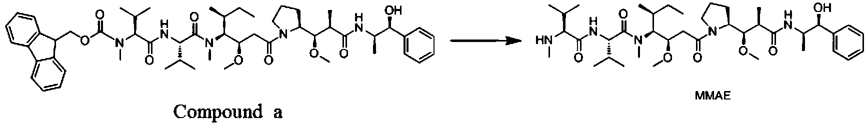

The Fmoc protecting group was removed from compound a by alkali method. Specifically, 2.0 g of compound a and 24 ml of acetonitrile were added to a three-necked flask, stirred and dissolved. 1.45 g of piperidine was then added for reaction.

After the reaction was completed, 40 ml of pure water was added to the reaction solution, and the reaction flask was placed in a low-temperature bath. The reaction solution was stirred at 0-5°C for 0.5 h and then filtered.

50 ml of dichloromethane was added for extraction. The organic phase was separated and concentrated to dryness under reduced pressure at 20-25°C to obtain 1.36 g of concentrate (i.e., crude MMAE).

The concentrate (i.e., crude MMAE) was dissolved in 20.4 ml of methylbenzene and transferred to a constant pressure dropping funnel.

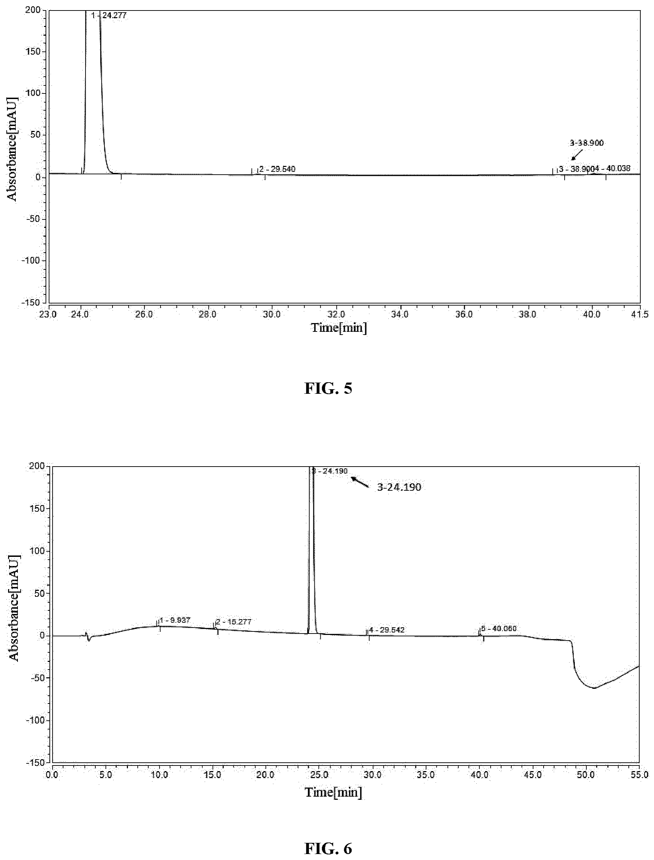

The solution of crude MMAE in methylbenzene was added dropwise to 61.2 ml of n-heptane under stirring, a large amount of solids precipitated, and filtration by suction was carried out to obtain 1.27 g of purified MMAE, with a yield of 83%, a purity of 99.90%, and a single impurity of 0.06%. The chromatogram is shown in FIG. 5.

### Example 6

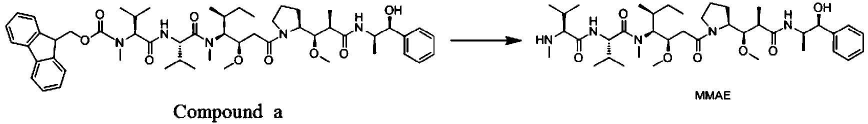

The Fmoc protecting group was removed from compound a by alkali method. Specifically, 2.0 g of compound a and 24 ml of acetonitrile were added to a three-necked flask, stirred and dissolved. 1.45 g of piperidine was then added for reaction.

After the reaction was completed, 20 ml of pure water was added to the reaction solution, and the reaction flask was placed in a low-temperature bath. The reaction solution was stirred at 0-5°C for 0.5 h and then filtered.

30 ml of dichloromethane was added for extraction. The organic phase was separated and concentrated to dryness under reduced pressure at 20-25°C to obtain 1.36 g of concentrate (i.e., crude MMAE).

The concentrate (i.e., crude MMAE) was dissolved in 13.6 ml of methylbenzene and transferred to a constant pressure dropping funnel.

The solution of crude MMAE in methylbenzene was added dropwise to 54.4 ml of n-heptane under stirring, a large amount of solids precipitated, and filtration by suction was carried out to obtain 1.29 g of purified MMAE, with a yield of 85%, a purity of 99.82%, and a single impurity of 0.11%. The chromatogram is shown in FIG. 6.

### Example 7

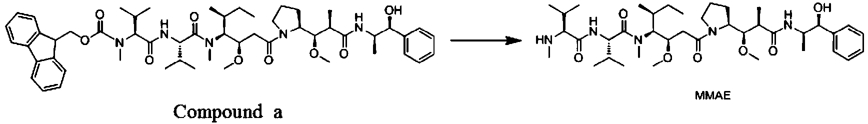

The Fmoc protecting group was removed from compound a by alkali method. Specifically, 2.0 g of compound a and 24 ml of acetonitrile were added to a three-necked flask, stirred and dissolved. 1.45 g of piperidine was then added for reaction.

After the reaction was completed, 20 ml of pure water was added to the reaction solution, and the reaction flask was placed in a low-temperature bath. The reaction solution was stirred at 0-5°C for 0.5 h and then filtered.

30 ml of dichloromethane was added for extraction. The organic phase was separated and concentrated to dryness under reduced pressure at 20-25°C to obtain 1.36 g of concentrate (i.e., crude MMAE).

The concentrate (i.e., crude MMAE) was dissolved in 13.6 ml of methylbenzene and transferred to a constant pressure dropping funnel.

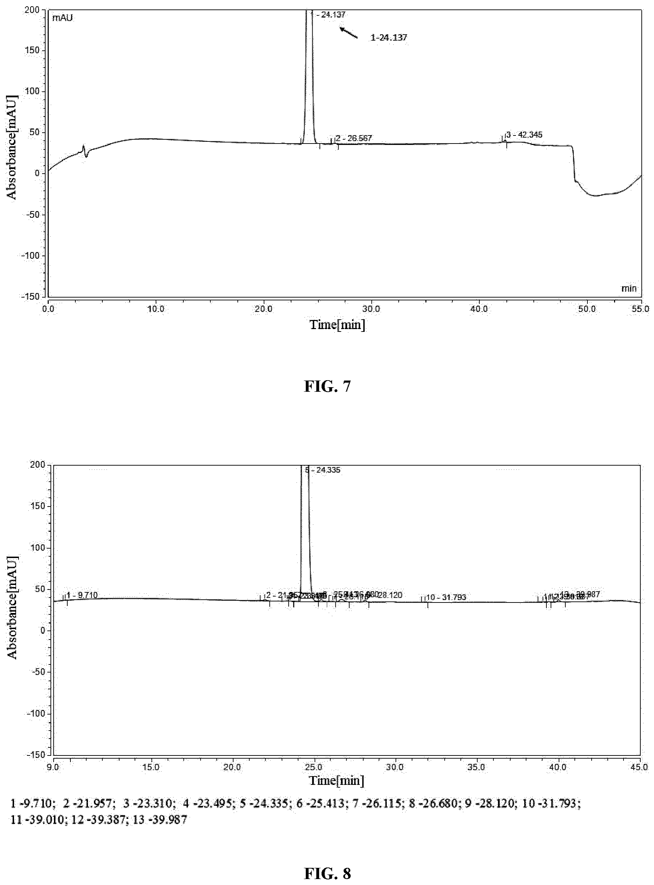

The solution of crude MMAE in methylbenzene was added dropwise to 47.6 ml of n-heptane under stirring, a large amount of solids precipitated, and filtration by suction was carried out to obtain 1.28 g of purified MMAE, with a yield of 84%, a purity of 99.87%, and a single impurity of 0.13%. The chromatogram is shown in FIG. 7.

### Example 8

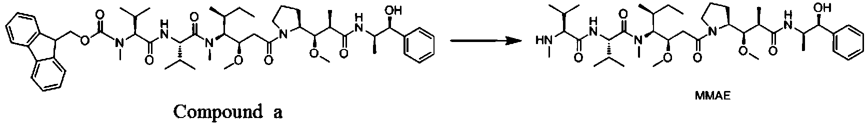

The Fmoc protecting group was removed from compound a by alkali method. Specifically, 1.0 g of compound a and 5 ml of ethanol were added to a three-necked flask, stirred and dissolved. 453 mg of piperidine was then added for reaction.

After the reaction was completed, 5 ml of pure water was added to the reaction solution, and the reaction flask was placed in a low-temperature bath. The reaction solution was stirred at 0-5°C for 0.5 h and then filtered.

10 ml of ethyl acetate was added for extraction. The organic phase was separated and concentrated to dryness under reduced pressure at 20-25°C to obtain 704 mg of concentrate (i.e., crude MMAE).

The concentrate (i.e., crude MMAE) was dissolved in 7 ml of ethyl acetate and transferred to a constant pressure dropping funnel.

The solution of crude MMAE in ethyl acetate was added dropwise to 21.1 ml of petroleum ether under stirring, a large amount of solids precipitated, and filtration by suction was carried out to obtain 634 mg of purified MMAE, with a yield of 83%, a purity of 99.11%, and a single impurity of 0.25%. Its chromatogram is shown in FIG. 8.

### Example 9

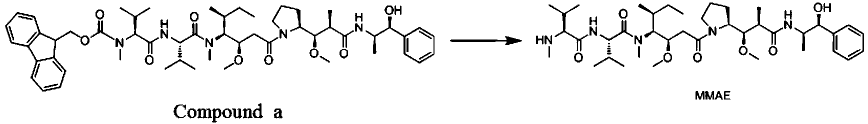

The Fmoc protecting group was removed from compound a by alkali method. Specifically, 1.0 g of compound a and 5 ml of acetonitrile were added to a three-necked flask, stirred and dissolved. 453 mg of piperidine was then added for reaction.

After the reaction was completed, 5 ml of pure water was added to the reaction solution, and the reaction flask was placed in a low-temperature bath. The reaction solution was stirred at 0-5°C for 0.5 h and then filtered.

10 ml of isopropyl acetate was added for extraction. The organic phase was separated and concentrated to dryness under reduced pressure at 20-25°C to obtain 639 mg of concentrate (i.e., crude MMAE).

The concentrate (i.e., crude MMAE) was dissolved in 6.4 ml of methylbenzene and transferred to a constant pressure dropping funnel.

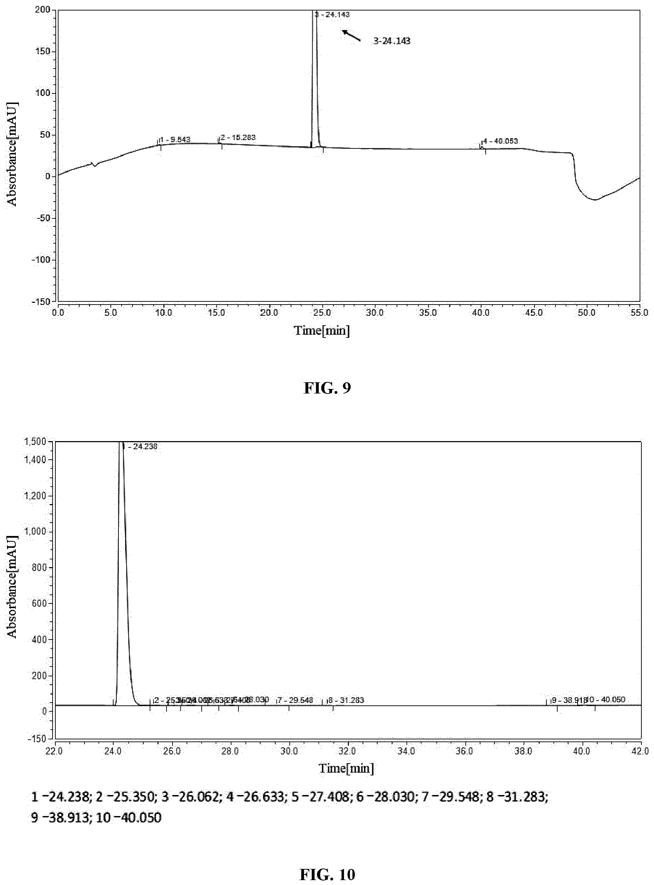

The solution of crude MMAE in methylbenzene was added dropwise to 19.2 ml of cyclohexane under stirring, a large amount of solids precipitated, and filtration by suction was carried out to obtain 588 mg of purified MMAE, with a yield of 77%, a purity of 99.85%, and a single impurity of 0.07%. The chromatogram is shown in FIG. 9.

### Example 10

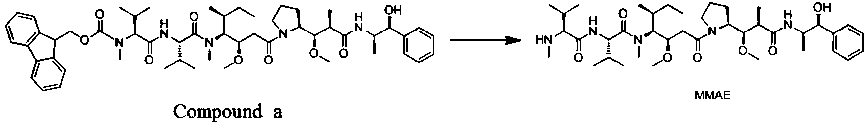

The Fmoc protecting group was removed from compound a by alkali method. Specifically, 1 g of compound a and 5 ml of acetonitrile were added to a three-necked flask, stirred and dissolved. 453 mg of piperidine was then added for reaction.

After the reaction was completed, 5 ml of pure water was added to the reaction solution, and the reaction flask was placed in a low-temperature bath. The reaction solution was stirred at 0-5°C for 0.5 h and then filtered.

10 ml of chloroform was added for extraction. The organic phase was separated and concentrated to dryness under reduced pressure at 20-25°C to obtain 653 mg of concentrate (i.e., crude MMAE).

The concentrate (i.e., crude MMAE) was dissolved in 6.5 ml of ethyl acetate and transferred to a constant pressure dropping funnel.

The solution of crude MMAE in ethyl acetate was added dropwise to 19.6 ml of n-pentane under stirring, a large amount of solids precipitated, and filtration by suction was carried out to obtain 596 mg of purified MMAE, with a yield of 78%, a purity of 99.63%, and a single impurity of 0.07%. The chromatogram is shown in FIG. 10.

### Example 11

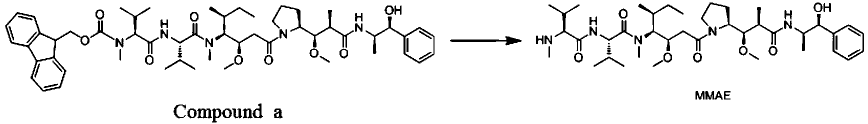

The Fmoc protecting group was removed from compound a by alkali method. Specifically, 1.0 g of compound a and 5 ml of acetonitrile were added to a three-necked flask, stirred and dissolved. 453 mg of piperidine was then added for reaction.

After the reaction was completed, 5 ml of pure water was added to the reaction solution, and the reaction flask was placed in a low-temperature bath. The reaction solution was stirred at 0-5°C for 0.5 h and then filtered.

10 ml of mlmethylbenzene was added for extraction. The organic phase was separated and concentrated to dryness under reduced pressure at 20-25°C to obtain 649 mg of concentrate (i.e., crude MMAE).

The concentrate (i.e., crude MMAE) was dissolved in 6.5 ml of acetone and transferred to a constant pressure dropping funnel.

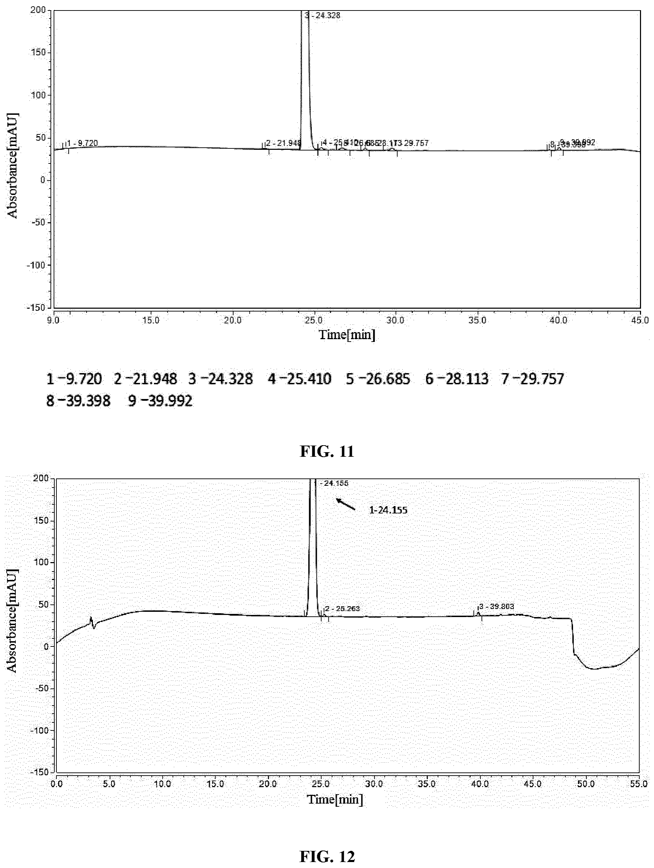

The solution of crude MMAE in acetone was added dropwise to 19.5 ml of methylcyclohexane under stirring, a large amount of solids precipitated, and filtration by suction was carried out to obtain 580 mg of purified MMAE, with a yield of 76%, a purity of 99.13%, and a single impurity of 0.21%. The chromatogram is shown in FIG. 11.

### Example 12

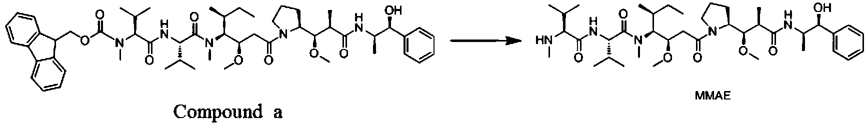

The Fmoc protecting group was removed from compound a by alkali method. Specifically, 1.0 g of compound a and 5 ml of acetonitrile were added to a three-necked flask, stirred and dissolved. 453 mg of piperidine was then added for reaction.

After the reaction was completed, 5 ml of pure water was added to the reaction solution, and the reaction flask was placed in a low-temperature bath. The reaction solution was stirred at 0-5°C for 0.5 h and then filtered.

10 ml of dichloromethane was added for extraction. The organic phase was separated and concentrated to dryness under reduced pressure at 20-25°C to obtain 693 mg of concentrate (i.e., crude MMAE).

The concentrate (i.e., crude MMAE) was dissolved in 6.9 ml of methylbenzene and transferred to a constant pressure dropping funnel.

The solution of crude MMAE in methylbenzene was added dropwise to 20.8 ml of methyl tert-butyl ether under stirring, a large amount of solids precipitated, and filtration by suction was carried out to obtain 603 mg of purified MMAE, with a yield of 79%, a purity of 99.77%, and a single impurity of 0.12%. The chromatogram is shown in FIG. 12.

### Example 13

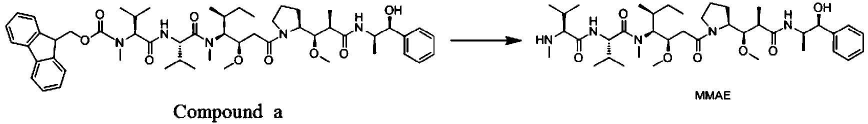

10.0 g of compound a and 50 ml of acetonitrile were added to a three-necked flask, stirred and dissolved. 2.71 g of piperidine was then added for reaction.

After the reaction was completed, 80 ml of pure water was added to the reaction solution, and the reaction flask was placed in a low-temperature bath. The reaction solution was stirred at 0-5°C for 0.5 h.

50 ml of chloroform was added for extraction. The organic phase was separated and concentrated to dryness under reduced pressure at 20-25°C to obtain 6.70 g of crude MMAE.

The crude MMAE was dissolved in 67 ml of methylbenzene and transferred to a constant pressure dropping funnel.

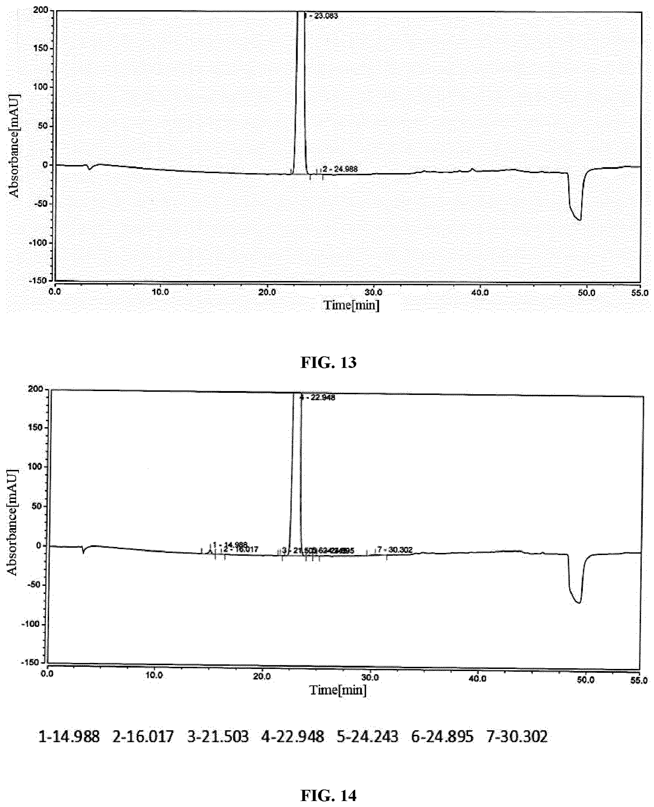

The solution of crude MMAE in methylbenzene was added dropwise to 201 ml of n-hexane under stirring, a large amount of solids precipitated, and filtration by suction was carried out to obtain 6.49 g of purified MMAE, with a yield of 85%, a purity of 99.96%, and a maximum single impurity of 0.04%. The chromatogram is shown in FIG. 13.

### Example 14

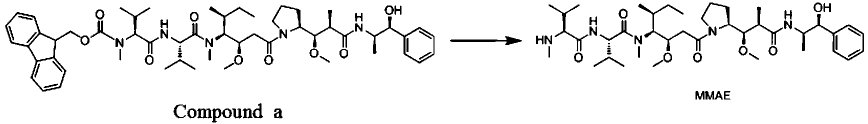

1.0 g of compound a and 5 ml of acetonitrile were added to a three-necked flask, stirred and dissolved. 271 mg of piperidine was then added for reaction.

After the reaction was completed, 10 ml of pure water was added to the reaction solution, and the reaction flask was placed in a low-temperature bath. The reaction solution was stirred at 0-5°C for 0.5 h.

15 ml of dichloromethane was added for extraction. The organic phase was separated and concentrated to dryness under reduced pressure at 20-25°C to obtain 683 mg of crude MMAE.

The crude MMAE was dissolved in 6.8 ml of acetone and transferred to a constant pressure dropping funnel.

The solution of crude MMAE in acetone was added dropwise to 20.5 ml of n-heptane under stirring, a large amount of solids precipitated, and filtration by suction was carried out to obtain 620 mg of purified MMAE, with a yield of 81%, a purity of 99.4%, and a maximum single impurity of 0.25%. The chromatogram is shown in FIG. 14.

### Example 15

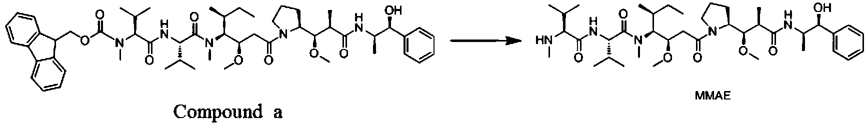

1200 mg of compound a and 10 ml of methanol were added to a three-necked flask, stirred and dissolved. 544 mg of piperidine was then added for reaction.

After the reaction was completed, 9.6 ml of pure water was added to the reaction solution, and the reaction flask was placed in a low-temperature bath. The reaction solution was stirred at 0-5°C for 0.5 h.

12 ml of methylbenzene was added for extraction. The organic phase was separated and concentrated to dryness under reduced pressure at 20-25°C to obtain 770 mg of crude MMAE.

The crude MMAE was dissolved in 11.6 ml of ethyl acetate and transferred to a constant pressure dropping funnel.

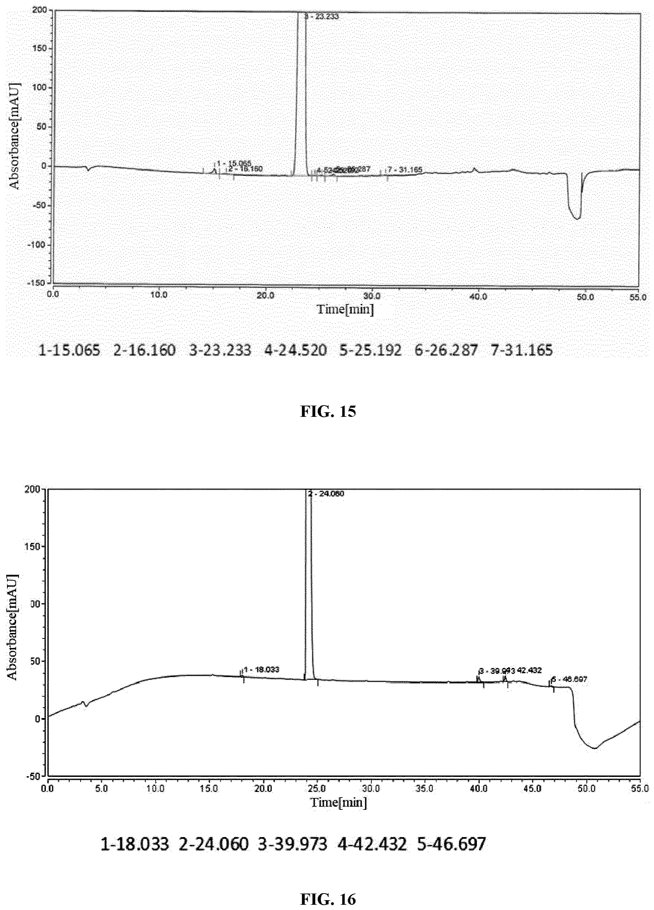

The solution of crude MMAE in ethyl acetate was added dropwise to 30.8 ml of n-hexane under stirring, a large amount of solids precipitated, and filtration by suction was carried out to obtain 724 mg of purified MMAE, with a yield of 79%, a purity of 99.20%, and a maximum single impurity of 0.38%. The chromatogram is shown in FIG. 15.

### Example 16

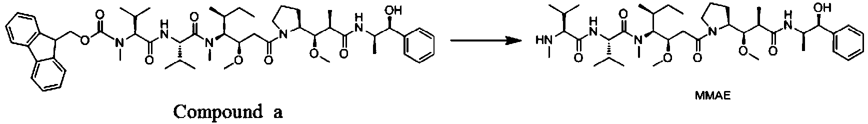

10.0 g of compound a and 100 ml of acetonitrile were added to a three-necked flask, stirred and dissolved. 9.05 g of piperidine was then added for reaction.

After the reaction was completed, 100 ml of pure water was added to the reaction solution, and the reaction flask was placed in a low-temperature bath. The reaction solution was stirred at 0-5°C for 0.5 h.

150 ml of dichloromethane was added for extraction. The organic phase was separated and concentrated to dryness under reduced pressure at 20-25°C to obtain 6.53 g of crude MMAE.

The crude MMAE was dissolved in 98 ml of methylbenzene and transferred to a constant pressure dropping funnel.

The solution of crude MMAE in methylbenzene was added dropwise to 294 ml of n-heptane under stirring, a large amount of solids precipitated, and filtration by suction was carried out to obtain 6.31 g of purified MMAE, with a yield of 82.6%, a purity of 99.53%, and a single impurity of 0.07%. The chromatogram is shown in FIG. 16.

### Comparative example 1

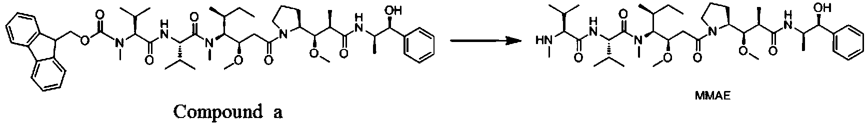

10.0 g of compound a and 70 ml of dichloromethane were added to a three-necked flask, stirred and dissolved. 3.1 g of diethylamine was then added for reaction.

After the reaction was completed, 100 ml of pure water and 70 ml of dichloromethane were added to the reaction solution for extraction. The organic phase was separated and concentrated to dryness under reduced pressure at 20-25°C to obtain 6.68 g of crude MMAE.

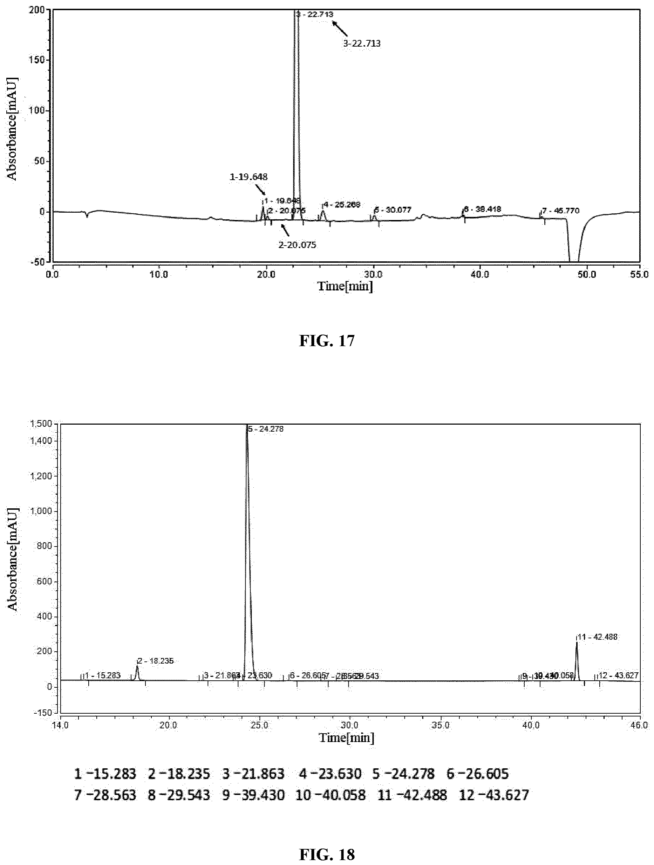

The crude MMAE was purified by column chromatography, and the solvent was evaporated to dryness to obtain 5.11g of purified MMAE, with a yield of 66.9%, a purity of 98.43% and a maximum impurity of 0.22%. The chromatogram is shown in FIG. 17.

### Comparative example 2

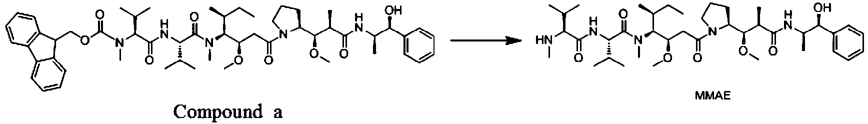

1.0 g of compound a and 10 ml of dichloromethane were added to a three-necked flask, stirred and dissolved. 485 mg of DBU was then added for reaction.

After the reaction was completed, the reaction solution was concentrated to dryness under reduced pressure at 20-25°C to obtain 1.52 g of crude MMAE.

The crude MMAE was dissolved in 15 ml of methanol and transferred to a constant pressure dropping funnel.

The solution of crude MMAE in methanol was added dropwise to 45.6 ml of methyl tert-butyl ether under stirring, solids precipitated, and filtration by suction was carried out to obtain 557 mg of purified MMAE, with a yield of 73%, a purity of 89.37%, and a single impurity of 7.10%. The chromatogram is shown in FIG. 18.

**Table 1 Comparison of yield and purity between the examples and comparative examples**

| Example | Yield | Purity |
|---|---|---|
| Example 1 | 88.5% | 99.97% |
| Example 2 | 83.7% | 99.6% |
| Example 3 | 85% | 99.58% |
| Example 4 | 86.9% | 99.21% |
| Example 5 | 83% | 99.9% |
| Example 6 | 85% | 99.82% |
| Example 7 | 84% | 99.87% |
| Example 8 | 83% | 99.11% |
| Example 9 | 77% | 99.85% |
| Example 10 | 78% | 99.63% |
| Example 11 | 76% | 99.13% |
| Example 12 | 79% | 99.77% |
| Example 13 | 85% | 99.96% |
| Example 14 | 81% | 99.44% |
| Example 15 | 79% | 99.2% |
| Example 16 | 82.6% | 99.53% |
| Comparative Example 1 | 66.9% | 98.43% |
| Comparative Example 2 | 73% | 89.37% |

As can be seen from Table 1, the method of producing and purifying MMAE provided by the present invention replaces the column chromatography adopted by methods in the prior art with the purification method of reverse dropwise addition for precipitation. The method can greatly improve the production efficiency and reduce the production cost. Most importantly, the method can significantly improve both the purity and yield of the product, which not only increases the purity of the final product from 98.43% to 99.97%, but also significantly increases the yield from 66.9% to 88.5%, greatly reducing production costs. In addition, the production and purification method provided by the present invention can produce MMAE with a good stability, and is suitable for scaled-up production. The method has unexpected technical effects.

The present invention has been exemplified by various specific examples. However, a person of ordinary skill in the art can understand that the present invention is not limited to each specific embodiment, and a person of ordinary skill can make various changes or variations within the scope of the present invention, and each technical feature mentioned in various places in this specification can be combined with each other without departing from the spirit and scope of the present invention. Such changes and variations are within the scope of the present invention.

## Claims

1. A method for producing and purifying a compound represented by formula (I) 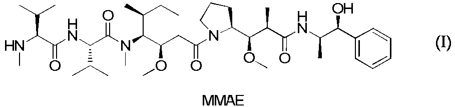 via a route of: 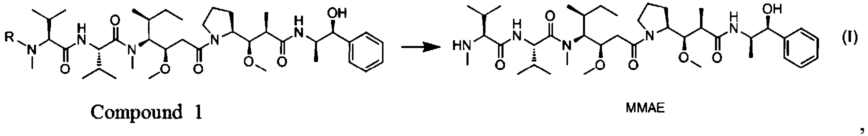 wherein R is an amino protecting group, and the method comprises steps of:
A. removing the amino protecting group R from compound 1 under alkaline condition,
B. completing a reaction in step A, then adding an appropriate amount of water to a reaction system of step A, stirring, filtering and collecting a filtrate,
C. adding an appropriate amount of a first organic solvent to the filtrate collected in step B, extracting and collecting an organic phase, concentrating the organic phase to obtain a concentrate a,
D. adding a second organic solvent to the concentrate a obtained in step C, dissolving to obtain a dissolved solution b, and
E. adding the dissolved solution b obtained in step D dropwise to a third organic solvent to allow precipitation of a large amount of solid, filtering by suction and collecting a filter cake to obtain MMAE;
wherein,
the amino protecting group R is selected from the group consisting of Fmoc protecting group and trifluoroacetyl;
the first organic solvent is selected from the group consisting of ethyl acetate, dichloromethane, isopropyl acetate, chloroform and methylbenzene;
the second organic solvent is selected from the group consisting of methylbenzene, ethyl acetate and acetone; and
the third organic solvent is selected from the group consisting of n-heptane, petroleum ether, n-hexane, cyclohexane, n-pentane, methylcyclohexane and methyl tert-butyl ether.

2. The method according to claim 1, wherein the compound 1 has a structure selected from the group consisting of compound a: 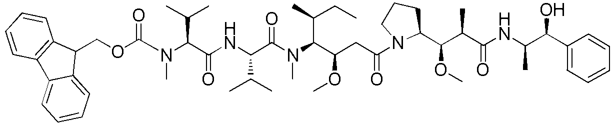 and compound b:

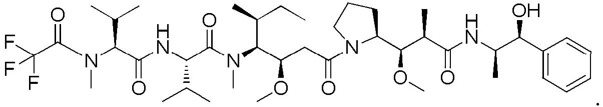

3. The method according to claim 1, wherein a weight-to-volume ratio (g/ml) of the compound 1 in step A to water in step B is 1:4 to 1:20, preferably 1:4 to 1:10; preferably, the stirring in step B is carried out at a low temperature, further preferably at a low temperature of 0°C to 10 °C.

4. The method according to claim 1, wherein a weight-to-volume ratio (g/ml) of the compound 1 in step A to the first organic solvent in step C is 1:5 to 1:25, preferably 1:5 to 1:15.

5. The method according to claim 1, wherein a weight-to-volume ratio (g/ml) of the concentrate a in step C to the second organic solvent in step D is 1:3 to 1:15, preferably 1:5 to 1:10.

6. The method according to claim 1, wherein a weight-to-volume ratio (g/ml) of the concentrate a in step C to the third organic solvent in step E is 1:15 to 1:45, preferably 1:20 to 1:40, preferably 1:25 to 1:35.

7. The method according to claim 1, wherein the removing the amino protecting group R from compound 1 under alkaline condition in step A is carried out by dissolving the compound 1 in an appropriate amount of a fourth organic solvent under stirring, and adding an alkali for reaction to remove the amino protecting group R; preferably, the fourth organic solvent is selected from the group consisting of acetonitrile, ethanol and methanol; further preferably, the alkali is selected from the group consisting of piperidine, diethylamine and DBU.

8. The method according to claim 7, wherein a weight-to-volume ratio (g/ml) of the compound 1 to the fourth organic solvent is 1:2 to 1:12, and/or an equivalence ratio of the compound 1 to the alkali is 1:2 to 1:8.

9. The method according to claim 1, wherein the water in step B is pure water, and/or the dissolving in step D is carried out under stirring.

10. Use of a reverse dropwise addition method in the production and purification of MMAE via a route of 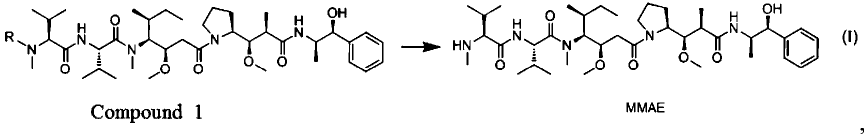 wherein R is an amino protecting group,
the reverse dropwise addition method comprises dissolving a reaction product produced by the route, which is to be purified and comprises MMAE, in a second organic solvent to obtain a solution, reversely adding the solution dropwise to a third organic solvent, filtering and drying to obtain purified MMAE;
wherein, the second organic solvent is selected from the group consisting of methylbenzene, ethyl acetate and acetone; and
the third organic solvent is selected from the group consisting of n-heptane, petroleum ether, n-hexane, cyclohexane, n-pentane, methylcyclohexane and methyl tert-butyl ether.

11. The use according to claim 10, wherein the reverse dropwise addition method comprises steps of:
(1) dissolving the reaction product comprising MMAE to be purified in a second organic solvent to obtain a solution 1,
(2) adding the solution 1 dropwise to a third organic solvent to allow precipitation of a large amount of solid, filtering by suction and collecting a filter cake to obtain purified MMAE.

12. The use according to claim 11, wherein a weight-to-volume ratio (g/ml) of the reaction product comprising MMAE to be purified in step (1) to the second organic solvent is 1:3 to 1:15, preferably 1:5 to 1:10.

13. The use according to claim 12, wherein a weight-to-volume ratio (g/ml) of the reaction product comprising MMAE to be purified in step (1) to the third organic solvent in step (2) is 1:15 to 1:45, preferably 1:20 to 1:40, preferably 1:25 to 1:35.
